# EUROPEAN PATENT APPLICATION

(11) **EP 2 769 679 A1**
(43) Date of publication of application: **27.08.2014**
(21) Application number: 12841845.6
(22) Date of filing: 17.10.2012
(51) Int. Cl.: A61B 17/00

(54) **FILTER DEVICE FOR CAPTURING EMBOLIC MATERIAL**

(30) Priority: 17.10.2011 JP 2011228194
(71) Applicant: Tokai Medical Products Inc., Aichi 486-0808 (JP)
(72) Inventor: TSUTSUI, Nobumasa, Kasugai-shi Aichi 486-0808 (JP); TSUTSUI, Yasuhiro, Kasugai-shi Aichi 486-0808 (JP); DAN, Kazunori, Kasugai-shi Aichi 486-0808 (JP); OKADA, Keiji, Kasugai-shi Aichi 486-0808 (JP)
(74) Representative: Zeuner Summerer Stütz
(86) International application number: PCT/JP2012/076778
(87) International publication number: WO 2013/058261

(57) **Abstract**

A filter device for capturing embolic material capable of retrogradely installing a filter is provided. A filter device for capturing embolic material 100 has a core wire 20 moving a filter member 40 to a target position in a blood vessel, a guide portion 30 formed on the distal front end side of the core wire 20 and guiding the core wire 20 in the blood vessel, the filter member 40 provided on the core wire 20 and capturing a blood clot and debris, and a sheath 50 capable of extending the core wire 20 therethrough.

## Description

### TECHNICAL FIELD

The present invention relates to a filter device for capturing embolic material.

### BACKGROUND ART

Conventionally, percutaneous angioplasty by placing a stent graft in an interventional operation has been performed in the narrowed portion in a blood vessel, such as a coronary artery, a carotid artery, and a venous graft. However, debris and a blood clot separated during the interventional operation may flow into the periphery of the blood vessel to clog the blood vessel.

In order to prevent embolic material in the blood vessel from flowing out to the periphery side in such an interventional operation, a filter device is installed on the periphery side from a portion to be treated to capture the embolic material. A filter device provided with a bag-like filter having a bag-like opening portion arranged on the proximal rear end side of a guide wire and a bag portion arranged on the distal front end side thereof is used as such a filter device (Patent Literature 1).

Such a conventional filter device is developed for protecting the terminal end in carotid artery stenting, and is antegradely inserted and placed into the blood vessel. Such a filter device is also effective for an interventional operation for an abdominal aorta and a lower limb artery.

### CITATION LIST

### PATENT LITERATURE

PTL 1: Japanese Patent Application Laid-Open (JP-A) No. 2005-537856

### SUMMARY OF INVENTION

### TECHNICAL PROBLEM

For instance, in an interventional operation which places a stent graft in a portion branched into a head and neck portion and an arm, such as an aortic arch, a blood clot adherent onto a portion to be placed and a heart valve may be scattered to the periphery side at the time of placing the stent graft. To prevent this scattering, it is necessary to install the filter device in a brachiocephalic artery or the like. In such a case, the conventional filter device is required to be antegradely inserted from a thigh. It is thus necessary to insert both a catheter placing the stent graft in the aortic arch and a guide wire installing the filter device in parallel from the thigh. Consequently, the filter device is made to pass through the portion to be treated so that the blood clot can be scattered at the time of insertion. In addition, the filter device which is located outside of the stent graft is difficult tobe withdrawn. Consequently, since actually it is impossible to install the filter device, the filter device cannot be installed in the above operation. Further, when the filter device is to be arranged antegradely in the lower limb interventional operation, since the blood vessel is severely narrowed and the narrowed portion is often long, it is difficult to make the filter device pass through the narrowed portion.

Accordingly, the present invention has been made in view of the above problems and a main object of the present invention is to provide a filter device for capturing embolic material capable of being arranged in a target position by retrogradely installing a filter to make the filter pass through a blood vessel which is entirely different from a blood vessel passed by a catheter placing a stent graft.

### SOLUTION TO PROBLEM

The present invention adopts the followingmeans to achieve the above object.

A filter device for capturing embolic material according to the present invention comprises a core wire, a guide portion provided on the distal front end of the core wire, a bag-like filter member provided on the core wire on the proximal rear end side of the guide portion to have an opening portion formed on the distal front end side and a bottom arranged on the proximal rear end side of the opening portion, and a sheath capable of accommodating therein the core wire and the filter member.

Such a filter device for capturing embolic material has the bag having the opening portion in the advancing direction introducing the core wire into a blood vessel and the bottom portion on the opposite side of the advancing direction. The filter device for capturing embolic material can thus be inserted from a carotid artery into a brachiocephalic artery or a left common carotid artery in an interventional operation placing a stent graft in an aortic arch. The filter device for capturing embolic material can thus be installed without passing through the aortic arch to be operated. The filter device for capturing embolic material can be retrogradely inserted even in the above operation which has not been able to install the filter device, and is not required to pass through a portion to be treated. The filter device for capturing embolic material can thus be easily installed on the periphery side of the portion to be treated. The filter device for capturing embolic material which does not pass through the portion to be treated can prevent a blood clot and debris in the aortic arch from being scattered at the time of inserting the filter device for capturing embolic material and of placing the stent graft. In addition, the filter device for capturing embolic material according to the present invention can accommodate the filter member in the sheath at the time of collecting debris and a blood clot captured in the filter member. The filter device for capturing embolic material can be withdrawn in a state that the filter member is accommodated in the sheath. The captured debris and blood clot can thus be prevented from being dropped. In addition, the filter device for capturing embolic material can be easily withdrawn without passing through the stent graft placed region. Further, the filter device can be inserted from the periphery side of the narrowed portion in a lower limb artery dilation operation without passing through the narrowed portion. The filter device can thus be easily arranged. In this specification and the claims, the term "distal front end side" is referred to as a side inserting the filter device for capturing embolic material. In addition, the term "proximal rear end side" is referred to as a side arranged on the hand side of the operator at the time of operating the filter device for capturing embolic material.

In the filter device for capturing embolic material according to the present invention, the bottom of the filter member may not be connected to the core wire. Typically, the bottom portion of the bag portion of the filter is often fixed onto the core wire. However, in the filter device for capturing embolic material according to the present invention, the bottom of the filter member may not be fixed onto the core wire. By adopting such a configuration, the opening portion of the filter member can be accommodated in the sheath before the bottom portion of the filter. The opening portion can thus be closed before the bag-like filter to prevent embolic material, such as debris and a blood clot, from being dropped from the opening.

In the filter device for capturing embolic material according to the present invention, an opening portion holding wire may be attached on the core wire and hold the filter member along the periphery of the opening portion. By adopting such a configuration, when the filter device for capturing embolic material is arranged in a target position to release the filter member from the sheath, the opening portion of the filter device can be easily opened and can remain open. The possibility that the opening portion is not completely opened in the blood vessel can thus be low. In addition, the opening portion presses the wall of the blood vessel to prevent a gap between the blood vessel and the opening portion. The possibility that embolic material, such as a blood clot and debris, flows from the gap to the periphery side can thus be low.

In the filter device for capturing embolic material according to the present invention, the opening portion may be tilted from a joint between the opening portion and the core wire to the distal front end side of the core wire. By adopting such a configuration, the opening portion can be easily pushed and tilted to the distal front end side of the sheath. By drawing the core wire into the sheath or pushing out the sheath, the opening portion can be easily accommodated in the sheath by a weaker force than when the opening portion is vertical or is tilted to the proximal rear end side.

In the filter device for capturing embolic material according to the present invention, a beam member may be extended between the opening portion holding wire and the core wire with respect to the opening portion. That is, the beam member holding the opening portion erected is extended between the opening portion holding wire and the core wire in the opening portion. A gap between the opening portion and the blood vessel caused by the opening portion which is accidentally tilted by a blood flow can thus be prevented.

The filter device for capturing embolic material according to the present invention may have a connecting portion connecting the beam member and the core wire, the connecting portion being slidable on the core wire, and a movement preventing member preventing the connecting portion from sliding to the proximal rear end side of the core wire from a specified position. By adopting such a configuration, the opening portion can be tilted to the distal front end side, but cannot be tilted to the proximal rear end side. The opening portion of the filter member can be prevented from being tilted to the proximal rear end side by a blood flow during an operation. In addition, the opening portion can be accommodated in the sheath even in the presence of the beam member since the connecting portion slides according to the tilting of the opening portion.

### ADVANTAGEOUS EFFECTS OF INVENTION

In the filter device for capturing embolic material according to the present invention, the filter device can be easily installed in the brachiocephalic artery without passing through the aorta in the interventional operation which places the stent graft in the portion branched into the head and neck portion and the arm, such as the aortic arch. In addition, the filter device for capturing embolic material can be withdrawn without passing through the stent graft. Further, in a lower limb artery dilation operation, the filter device for capturing embolic material can be inserted from the periphery side of a narrowed portion without passing through the narrowed portion.

### BRIEF DESCRIPTION OF DRAWINGS

Fig. 1 is a schematic diagram showing the configuration of a filter device for capturing embolic material 100 according to an embodiment.
Fig. 2 is an enlarged view of the main configuration of the filter device for capturing embolic material 100 according to the embodiment.
Fig. 3 is a perspective view showing only a core wire 20 and an opening portion holding wire 43 according to the embodiment.
Figs. 4A and 4B are enlarged views showing a connecting portion of the opening portion holding wire 43 and a beam member 44 according to the embodiment.
Fig. 5 is a partially perspective sectional view showing the state of the filter device for capturing embolic material 100 according to the embodiment before use.
Figs. 6A to 6D are perspective views showing a method of withdrawing the filter device for capturing embolic material 100 according to the embodiment.

### DESCRIPTION OF EMBODIMENT

### (Embodiment)

Hereinafter, a filter device for capturing embolic material 100 according to an embodiment will be described in detail.

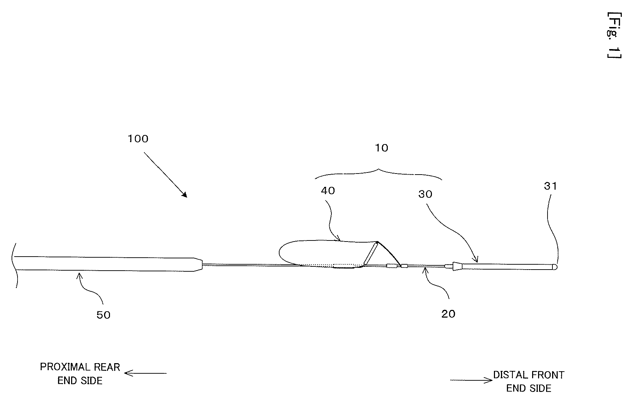

Fig. 1 is a schematic diagram of the filter device for capturing embolic material 100 according to the embodiment. The filter device for capturing embolic material 100 according to the embodiment mainly has a core wire 20 moving a filter member 40 to the target position in a blood vessel, a guide portion 30 formed on the distal front end side of the core wire 20 and guiding the core wire 20 in the blood vessel, the filter member 40 provided on the core wire 20 and capturing a blood clot and debris, and a sheath 50 capable of accommodating therein the core wire 20 and the filter member.

The core wire 20 is mainly used for arranging the filter member 40 in a desired blood vessel (e.g., a brachiocephalic artery) when the operator operates the core wire in his/her hand and for withdrawing the filter member 40 after a stent graft is placed in an interventional operation.

The core wire 20 is made of Teflon (trademark) coated stainless steel, and is tapered to be thinner toward the distal front end side, as shown in Fig. 1. This is for forming the distal front end side more flexibly to enhance following capability in the blood vessel.

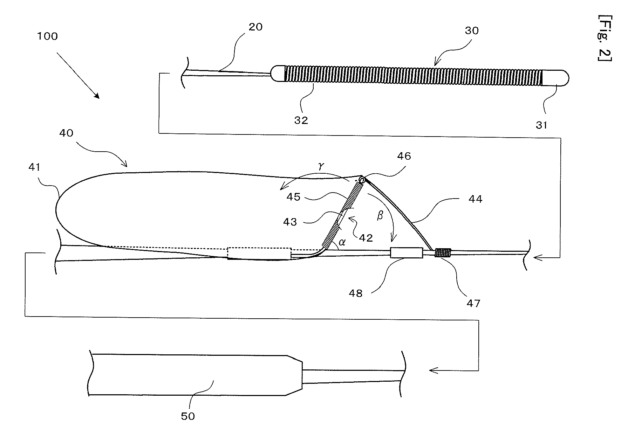

As shown in Fig. 2, the guide portion 30 has a distal front end chip 31 provided on the distal front end side of the core wire, and a marker member 32 provided on the distal front end side of the core wire 20 to surround the core wire 20. The distal front end chip 31 is silver soldered, and has a distal front end with a curved surface. The distal front end chip 31 prevents the blood vessel from being damaged when the core wire 20 is advanced thereinto.

The marker member 32 has a wire made of a platinum-iridium alloy as a radiation non-transmission material around the guide portion 30. The position of the marker member 32 can be identified on an X-ray transmission screen during treatment. The marker member 32 helps the operator to identify the position of the distal front end of the core wire 20.

The filter member 40 has a bag-like filter 41, an opening portion holding wire 43 provided in an opening portion 42 of the filter 41 and maintaining the opening portion 42 opened, and a beam member 44 holding the opening portion holding wire 43 erected.

The filter 41 is made of polyurethane as a biocompatible material, and formed with a large number of small holes of 100 µm to make blood pass therethrough but to capture a blood clot and debris. The filter 41 is formed in a bag shape with the opening portion 42 in which one side thereof is opened. The filter 41 captures embolic material, such as a blood clot and debris, from the opening portion 42.

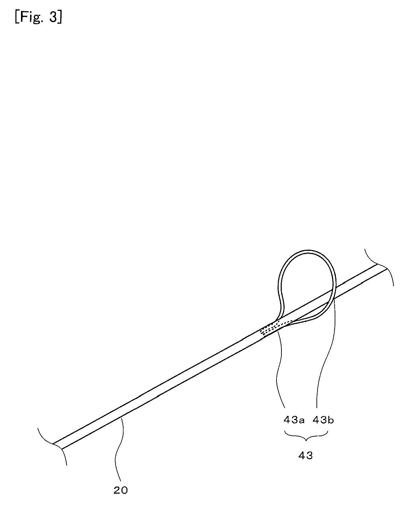

To form the opening portion holding wire 43, one wire is folded back to make its distal front end circular, as shown in Fig. 3, so that angle a (see Fig. 2) formed between the axis of a straight portion 43a at the base and the plane of a circular portion 43b is approximately 45° to 80°. That is, the straight portion 43a at the base is parallel with the core wire 20, and the circular portion 43b of the opening portion holding wire 43 holds the angle of approximately 60° with respect to the core wire 20. The opening portion holding wire 43 has flexibility to some extent. Atangle a formed between the axis of the straight portion 43a at the base and the plane of the circular portion 43b, the opening portion holding wire 43 can be tilted by giving a force to some extent. The circular portion 43b can be deformed in an elliptical shape or an elliptical shape very close to a straight line, and can be returned to the form shown in Fig. 3 by releasing the force. The opening portion 42 of the filter 41 is provided along the opening portion holding wire 43. The form of the opening of the filter 41 is changed according to the changed form of the opening portion holding wire. As shown in Fig. 2, a marker coil 45 is helically wound around the circular portion 43b of the opening portion holding wire 43. By the marker coil 45, the positions of the opening portion holding wire 43 and the opening portion 42 of the filter member 40 can be identified on the X-ray transmission screen during treatment.

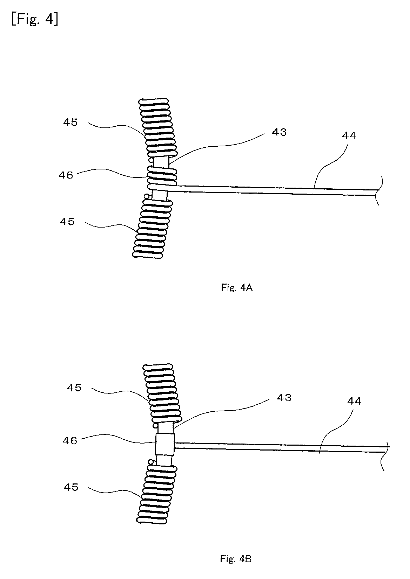

The beam member 44 is extended between the farthest position of the circular portion 43b of the opening portion holding wire 43 from the core wire 20, that is, the highest position in Fig. 2 (referred to as a "far position") and the core wire 20 at the distal front end side from the opening portion 42. A connecting portion 46 of the beam member 44 and the opening portion holding wire 43 is provided in the far position of the opening portion holding wire 43, and is rotatable with respect to the opening portion holding wire 43. The method of the connecting portion 46 connecting the beam member 44 and the rotation holding wire 43 is not limited as long as the connecting portion 46 is rotatable with the opening portion holding wire 43. As shown in Fig. 4A, for instance, the end of the beam member 44 may be wound around the rotation holding wire. In addition, as shown in Fig. 4B, the end of the beam member 44 of the connecting portion 46 may be tubular to have clearance with respect to the opening portion holding wire 43 to make the opening portion holding wire 43 pass into the tubular end, thereby making rotatable. As shown in Fig. 2, a connecting portion 47 of the core wire 20 and the beam member 44 is wound to be slidable with respect to the axis of the core wire 20. Obviously, when the connecting portion 47 of the core wire 20 and the beam member 44 should be slidable, its configuration is not limited. The wound portion of the beam member may be tubular to be slidable with respect to the core wire 20. The connecting portion 47 slides to the front end side so as not to inhibit the tilting of the circular portion 43b of the opening portion holding wire 43 to the distal front end side (direction β). A stopper 48 is provided on the proximal rear end side of the connecting portion 47 so as not to slide the connecting portion 47 to the proximal rear end side. The stopper 48 which inhibits the connecting portion 47 of the beam member can prevent the circular portion 43b from being tilted to the proximal rear end side (direction γ) above a certain level. With this, the opening portion holding wire 43 which is installed in the blood vessel can be prevented from being tilted to the proximal rear end side of the core wire 20 by a blood flow.

The sheath 50 made of polyurethane is tubular, and has flexibility. The sheath 50 is formed with a lumen extending therethrough along the overall length thereof. The lumen on the distal front end side of the sheath 50 is formed in a size which can accommodate therein the core wire 20 and the filter member 40, and can relatively move the core wire 20 in the sheath 50. The diameter of the sheath 50 may be different at the time of inserting the filter device for capturing embolic material 100 into a body and of withdrawing the filter device for capturing embolic material 100 therefrom. Specifically, for insertion, a relatively thin sheath is used to make the filter device for capturing embolic material 100 pass through the blood vessel more easily. In addition, for withdrawal, a relatively thick sheath is used to withdraw thereinto the opening portion holding wire etc. which are once opened.

A method of using the filter device for capturing embolic material 100 formed as described above according to the present invention will be described by taking as an example, the case of installing the filter member 40 in the brachiocephalic artery in the interventional operation which places the stent graft in a portion branched into a head and neck portion and an arm, such as an aortic arch.

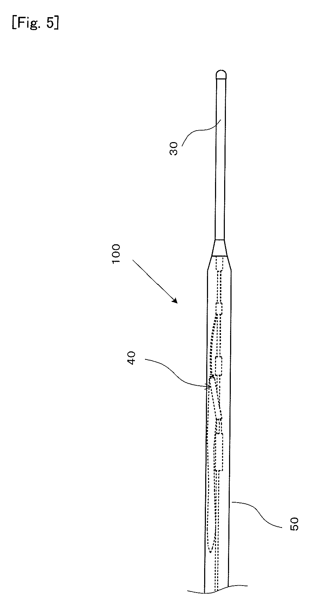

As shown in Fig. 5, the filter device for capturing embolic material 100 which accommodates the core wire 20 and the filter member 40 in the sheath 50 is prepared. A puncturing needle is punctured into a carotid artery. Thereafter, the core wire 20 is inserted into the blood vessel with the guide portion 30 on the distal front end side. While the guide portion 30 is moved along the blood vessel, the core wire 20 is slid and inserted into the blood vessel. While the positions of the guide portion 30 and the opening portion 42 are identified on the X-ray transmission screen, the filter member 40 is moved to a target position in the brachiocephalic artery. The sheath 50 is drawn out by a predetermined distance to push out the filter member 40 from the distal front end side of the sheath 50. With this, the opening portion holding wire 43 of the filter member 40 is released, the circular portion 42b of the opening portion holding wire 43 is opened, and the opening portion 42 of the filter member 40 is opened circularly. The filter member 40 is arranged in the state in Fig. 1 in the target position in the brachiocephalic artery. In spite of being inserted from the carotid artery, the opening of the filter member 40 is on the aortic arch side and the bottom portion thereof is on the opposite side of the aortic arch side. The sheath 50 is then withdrawn.

A catheter placing the stent graft is inserted from an artery superficial portion in a thigh to place the stent graft. A blood clot and debris which are separated in such treatment are captured by the arranged filter 41.

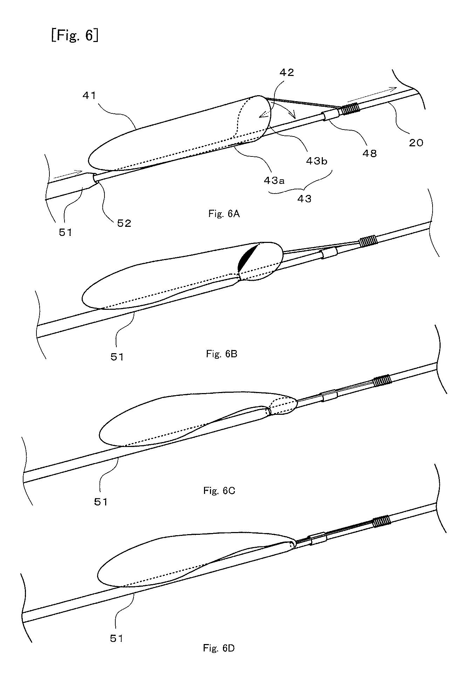

After the placing of the stent graft is completed, the catheter placing the stent graft is removed. Then, the filter device for capturing embolic material 100 is withdrawn by the following method. To withdraw the filter device for capturing embolic material 100, a sheath 51 for withdrawal is inserted from the proximal rear end side of the core wire exposed to the outside of the body. As shown in Fig. 6, the sheath 51 is inserted to the present portion of the filter member 40. The sheath 51 for withdrawal has a slightly larger opening 52 on the distal front end side than the sheath 50 for insertion. The core wire 20 on the proximal rear end side exposed to the outside of the body is drawn into the sheath 51 or the sheath 51 is pushed out. As shown in Figs. 6A to 6D, the opening 52 on the distal front end of the sheath 51 is moved to the base of the opening portion 42. The straight portion 43a of the opening portion holding wire 43 is then drawn into the sheath 51 (Fig. 6B). At this time, since the bottom of the filter 41 is not provided on the core wire 20, the filter 41 is not accommodated in the sheath 51. While the straight portion 43a of the opening portion holding wire 43 is drawn into the sheath 51 in this state, the circular portion 43b of the opening portion holding wire 43 is tilted to reduce the opening area thereof and drawn into the sheath 51 (Figs. 6B and 6C) . Finally, the entire opening portion holding wire 43 is drawn into the sheath 51 (Fig. 6D). When the opening portion holding wire 43 is tilted, the beam member 44 is slid to the distal front end side in the connecting portion of the core wire 20 and the beam member 44, so that the tilting of the opening portion holding wire 43 cannot be inhibited. In a state where the entire opening portion holding wire 43 is accommodated in the sheath 51, the opening portion 42 is completely closed to prevent a blood clot and debris from leaking out from the inside of the filter 41. In this state, the filter device for capturing embolic material 100 is withdrawn to the outside of the body. Further, after the core wire 20 is drawn to draw the entire filter 41 into the sheath 51, the filter device for capturing embolic material 100 may be withdrawn. The interventional operation is thus ended.

According to the filter device for capturing embolic material 100 according to the present invention, the filter 41 is arranged in the target position in the brachiocephalic artery without passing through the aortic arch. Accordingly, the filter device for capturing embolic material 100 can prevent a blood clot and debris in the stent graft placed region of the aortic arch from being scattered. Also, the filter device for capturing embolic material 100 is removed without pas sing through the portion to be treated. The filter device for capturing embolic material 100 can thus be easily withdrawn without the installed stent graft inhibiting removal.

The present invention is not limited to the embodiment, and can be embodied in various forms as long as they belong to the technical range of the present invention.

For instance, in the embodiment, as the material of the core wire 20, stainless steel is used. However, the present invention is not limited to this. Metals, such as a nickel-titanium alloy and a nickel-titanium-cobalt alloy, can also be used. In addition, the entire core wire 20 is not always made of the same material, and may be made of a different material according to portion to give different rigidity and flexibility according to portion.

In the above embodiment, the core wire 20 is tapered to be thinner toward the distal front end side. However, the present invention is not limited to this. The core wire 20 may be a wire having a uniform thickness.

In the embodiment, as the material of the marker member 32, a platinum-iridium alloy is used. However, the present invention is not limited to this. Gold, platinum, and other radiopaque material can also be used. In addition, the entire marker member is not required to be made of the radiopaque material, and may be partially made of the radiopaque material.

In the above embodiment, as the sheath 50, polyurethane is used. However, the present invention is not limited to this. For instance, a fluoride resin and nylon etc. can be used.

In the embodiment, as the material of the filter, polyurethane is used. However, the present invention is not limited to this. Silicon may be used.

In the embodiment, the filter member 40 is installed in the brachiocephalic artery in the interventional operation which places the stent graft in the portion branched into the head and neck portion and the arm, such as the aortic arch. However, in the treatment of blood vessel narrowing in the thigh, the filter device for capturing embolic material 100 can also be retrogradely inserted from the artery superficial portion of a knee and an ankle. The filter device for capturing embolic material 100 which is antegradely inserted is required to pass through a portion to be treated. Consequently, it is difficult to make the filter device itself pass through the narrowed portion. However, the filter device for capturing embolic material 100 according to the present invention can be installed without passing through the portion to be treated.

### INDUSTRIAL APPLICABILITY

As described in the embodiment, the present invention can be used for the interventional operation, in particular, for preventing a blood clot and debris from being scattered.

### REFERENCE SIGNS LIST

20 ... core wire, 30 ... guide portion, 31 ... distal front end chip, 32 ... marker member, 40 ... filter member, 41 ... filter, 42 ... opening portion, 42b ... circular portion, 43 ... opening portion holding wire, 43a ... straight portion, 43b ... circular portion, 44 ... beam member, 45 ... marker coil, 46 ... connecting portion, 47 ... connecting portion, 48 ... stopper, 50 ... sheath, 51 ... sheath, 52 ... opening, 100 ... filter device for capturing embolic material

## Claims

1. A filter device for capturing embolic material comprising:
a core wire;
a guide portion provided on the distal front end side of the core wire;
a bag-like filter member provided on the core wire on the proximal rear end side of the guide portion to have an opening portion formed on the distal front end side and a bottom arranged on the proximal rear end side of the opening portion; and
a sheath capable of accommodating therein the core wire and the filter member.

2. The filter device for capturing embolic material according to claim 1, wherein the bottom of the filter member is not connected to the core wire.

3. The filter device for capturing embolic material according to any one of claims 1 and 2, wherein an opening portion holding wire is provided on the core wire and holds the filter member along the periphery of the opening portion.

4. The filter device for capturing embolic material according to any one of claims 1 to 3, wherein the opening portion is tilted from a joint between the opening portion and the core wire to the distal front end side of the core wire.

5. The filter device for capturing embolic material according to any one of claims 1 to 4, wherein a beam member is extended between the opening portion holding wire and the core wire with respect to the opening portion.

6. The filter device for capturing embolic material according to claim 5, further comprising a connecting portion connecting the beam member and the core wire, the connecting portion being slidable on the core wire, and a movement preventing member preventing the connecting portion from being slid to the proximal rear end side of the core wire from a specified position.

7. The filter device for capturing embolic material according to any one of claims 1 to 6, further comprising a withdrawing sheath used for withdrawal.
